# FASCICULE DE BREVET EUROPEEN

(11) **EP 3 887 493 B1**
(45) Date de publication et mention de la délivrance du brevet: **15.01.2025**
(21) Numéro de dépôt: 19868174.4
(22) Date de dépôt: 25.11.2019
(51) Int. Cl.: A01N 25/30, C11D 1/90, A01N 37/44, C07C 237/06, C08K 5/19, C11D 3/48, A61K 8/44, A61Q 5/02, A61Q 5/12, A61Q 19/10, C07C 227/18

(54) **PROCÉDÉ DE PRÉPARATION D'UNE COMPOSITION TENSIOACTIVE À BASE DE SEL D'ESTER DE GLYCINE BÉTAÏNE ET COMPOSITION AINSI OBTENUE**
VERFAHREN ZUR HERSTELLUNG EINER TENSIDZUSAMMENSETZUNG AUF DER BASIS EINES GLYCINBETAINESTERSALZES UND SO ERHALTENE ZUSAMMENSETZUNG
PROCESS FOR PREPARING A SURFACTANT COMPOSITION BASED ON A GLYCINE BETAINE ESTER SALT, AND COMPOSITION THUS OBTAINED

(30) Priorité: 27.11.2018 FR 1871892
(43) Date de publication de la demande: 06.10.2021
(73) Titulaire: Surfactgreen, 60201 Compiegne Cedex (FR)
(72) Inventeur: PESSEL, Freddy, 35708 RENNES CEDEX 7 (FR); GALLE, Francis, 35708 RENNES CEDEX 7 (FR); DIVET, Pierre-Yves, 92200 NEUILLY-SUR-SEINE (FR); ROUSSEL, Xavier, 72000 LE MANS (FR)
(74) Mandataire: Renard, Emmanuelle
(86) Numéro de dépôt international: PCT/FR2019/052801
(87) Numéro de publication internationale: WO 2020/109710

(56) Documents cités:
- WO-A1-2015/003968
- WO-A1-2015/078890
- WO-A2-2009/150369
- F. GOURSAUD ET AL., GREEN CHEM., vol. 10, 2008, pages 310 - 320, XP055531793

## Description

### OBJET DE L'INVENTION

La présente invention concerne un procédé de préparation d'une composition tensioactive à base de sel d'ester de glycine bétaïne, ainsi que la composition ainsi obtenue. Elle porte également sur les utilisations de cette composition. Elle a en outre pour objet un produit comprenant la composition précitée.

### ARRIERE-PLAN DE L'INVENTION

Les tensioactifs constituent des matières premières indispensables à la fabrication d'une diversité de produits. Parmi ceux-ci, les tensioactifs cationiques représentent, certes, un marché moins étendu que celui des tensioactifs anioniques ou non ioniques, mais ils présentent toutefois un intérêt dans de multiples applications, notamment dans la fabrication de produits détergents et cosmétiques, ainsi que dans le traitement de l'eau.

En raison de leur toxicité, certains tensioactifs tel que les sels de diméthyldialkylammonium, présents dans la plupart des adoucissants textiles, voient leur utilisation limitée, voire abandonnée, dans certains pays européens comme l'Allemagne et les Pays-Bas. Sous la pression de l'écologie, les producteurs de tensioactifs doivent proposer des produits moins polluants, plus biodégradables et présentant une écotoxicité la plus faible possible. Aux contraintes environnementales s'ajoute le souhait pour les consommateurs de disposer de produits les plus naturels possibles.

Dans ce contexte, la glycine bétaïne, substance naturelle peu onéreuse, constitue une matière première de choix pour la préparation d'agents tensioactifs. Issue de la mélasse de la betterave à sucre, obtenue après extraction du saccharose, elle reste actuellement un sous-produit de l'industrie sucrière. Le greffage sur la glycine bétaïne d'alcools et d'amines gras permet d'accéder à des molécules amphiphiles cationiques sans l'étape classique de quaternisation d'une amine tertiaire à l'aide d'agents de méthylation généralement toxiques.

Il a ainsi été proposé dans le brevet US-7,829,521 des esters de glycine bétaïne obtenus par réaction de la glycine bétaïne avec au moins deux équivalents d'un acide sulfonique, tel que l'acide méthanesulfonique, et un alcool comprenant au moins 18 atomes de carbone. Les tensioactifs cationiques ainsi obtenus sont présents en mélange avec les réactifs résiduels et ce mélange peut éventuellement être enrichi en ester de glycine bétaïne par traitement à l'aide d'un solvant organique et/ou purifié sur colonne de gel de silice. Il est ainsi possible d'obtenir un ester d'octadécyle ou d'octadécényle de glycine bétaïne avec un rendement d'environ 70% ou 85%, respectivement. Ceux-ci présentent une tension de surface d'environ 37 mN/m et sont plus particulièrement destinés à une application cosmétique.

D'autres tensioactifs similaires et également à chaîne plus courte (ester laurique) sont présentés dans la publication de F. Goursaud et al dans Green Chem., 2008, 10, 310-320. Ces tensioactifs sont eux aussi préparés en présence d'une quantité d'acide méthanesulfonique d'au moins deux équivalents, par rapport à la quantité de glycine bétaïne mise en oeuvre, qui est décrite comme étant nécessaire à l'obtention d'un rendement acceptable (d'au moins 70%). La tension de surface des esters obtenus après purification est, là encore, très élevée (de 32 à 37 mN/m).

L'ester laurique de glycine bétaïne est également décrit, seul ou en mélange avec l'ester myristique correspondant, dans la demande de brevet WO 2013/188508. Dans ce document, il est démontré (Tableau 5) que le mélange réactionnel brut présente une tension de surface plus faible que l'ester de glycine bétaïne pur, permettant d'envisager son utilisation dans des détergents ménagers. Ce mélange réactionnel renferme dans tous les cas moins de 65% en poids d'ester de glycine bétaïne et/ou plus de 20% en poids d'acide sulfonique résiduel (Tableau 4). Or, il a été démontré par les présents inventeurs que l'ajustement de ces deux paramètres permettait d'abaisser encore la tension superficielle de ces mélanges.

De son côté, le document WO 2015/078890 divulgue une composition tensioactive obtenue en faisant réagir, dans une première étape, de la glycine bétaïne avec un alcool de formule R1-OH renfermant de 1 à 6 atomes de carbone, puis avec un alcool de formule R2-OH à plus longue chaîne, en présence d'un hémiacétal de sucre. Ce procédé en deux étapes résulte une composition tensioactive complexe.

Par ailleurs, le document WO 2015/003968 décrit des actifs déodorants constitués de sels d'ester de glycine bétaïne, qui peuvent être préparés suivant un procédé impliquant la réaction de la glycine bétaïne avec un alcool R-OH où R est un alkyle en C2-C22, en présence d'un excès d'acide, à une température comprise entre 60 et 160°C. Seuls les Exemples 1 et 2 concernent des esters en C8-C22 donc possiblement dotés de propriétés tensioactives. Dans ces exemples, la température de réaction est de 140°C et le taux d'acide est inférieur à 1,5 équivalent. Les esters de glycine bétaïne sont obtenus avec un rendement ne dépassant pas 44%.

Après de longues recherches, les inventeurs ont ainsi mis au point un procédé modifié de préparation de sels d'ester de glycine bétaïne dans lequel la réaction d'estérification est réalisée en présence d'une quantité réduite d'acide et à plus haute température que les procédés de l'art antérieur décrits ci-dessus. Ce procédé permet d'obtenir en une seule étape des compositions tensioactives présentant des tensions de surface plus faibles et éventuellement une proportion plus élevée d'ester de glycine bétaïne que les compositions tensioactives décrites dans l'art antérieur, sans affecter sensiblement le rendement de la réaction. Ce procédé peut en outre être mis en oeuvre en un temps plus court et ne nécessite pas d'étape de purification du produit obtenu.

Le procédé de synthèse mis en oeuvre selon l'invention est simple, efficace, respectueux de l'environnement, sans solvant ni rejet polluant, et facilement transposable à l'échelle industrielle pour obtenir de manière reproductible une composition tensioactive particulièrement performante.

### RESUME DE L'INVENTION

L'invention a pour objet un procédé de préparation d'une composition tensioactive, comprenant les étapes successives consistant à :
(1) faire réagir de la glycine bétaïne ou l'un de ses sels avec un alcool gras linéaire ou ramifié, saturé ou insaturé, renfermant de 8 à 24 atomes de carbone, en présence d'un acide organique ou inorganique ;
(2) refroidir le milieu réactionnel à une température de 20 à 90°C ; et
(3) récupérer la composition tensioactive ainsi obtenue,
caractérisé en ce que l'acide organique ou inorganique représente de 1,5 à 1,9 équivalent molaire par rapport au nombre de moles de glycine bétaïne et en ce que la réaction est effectuée à une température de 150 à 180°C.

Elle a également pour objet la composition tensioactive obtenue suivant ce procédé, renfermant les constituants suivants :
(a) de 65 à 85% en poids, de préférence de 70 à 80% en poids, de sel d'ester de glycine bétaïne,
(b) de 1 à 20% en poids, par exemple de 1 à 9% en poids ou de 10 à 20% en poids, d'alcool gras,
(c) de 1 à 20% en poids, par exemple de 5 à 15% en poids d'acide organique ou inorganique, et
(d) de 1 à 20% en poids, par exemple de 2 à 15% en poids, de sel de glycine bétaïne, par rapport au poids total de ces quatre constituants.

L'invention a encore pour objet l'utilisation de la composition tensioactive précitée comme agent mouillant, dispersant de particules, inhibiteur de corrosion, solubilisant, antistatique et/ou démêlant et/ou pour améliorer l'effet et/ou la rémanence de substances insecticides et/ou pour améliorer le pouvoir désinfectant et/ou la rémanence de l'effet désinfectant de substances antimicrobiennes.

Elle a également pour objet l'utilisation de cette composition pour la fabrication de plastiques ou de produits destinés :
- au traitement et/ou au nettoyage du corps, de plantes ou de surfaces dures, en particulier de produits cosmétiques, de produits de lavage de véhicules, de produits ménagers, de produits de nettoyage industriel, de produits d'ensimage de fibres et de produits phytosanitaires ;
- au traitement de l'eau ;
- au revêtement de routes ; ou
- à l'extraction du pétrole.

Elle a en outre pour objet un produit comprenant la composition précitée et au moins un constituant choisi parmi : (a) les tensioactifs anioniques, les tensioactifs non ioniques et leurs mélanges, (b) les agents antimicrobiens, (c) les insecticides, et leurs mélanges.

Outre sa faible tension de surface, la composition tensioactive obtenue selon l'invention présente l'avantage d'être biodégradable (selon la norme OECD 301), faiblement toxique pour l'environnement (suivant les normes OECD 201 et 202) et pour l'homme.

### DESCRIPTION DETAILLEE

### Procédé

La composition tensioactive selon l'invention peut être préparée suivant un procédé d'estérification de la glycine bétaïne, ou triméthylglycine. La glycine bétaïne peut être d'origine végétale ou synthétique. Il est nécessaire de la protoner préalablement à l'aide d'un acide organique ou inorganique, dans la mesure où elle se présente sous forme zwitterionique (présence d'une fonction carboxylate). L'acide peut notamment être choisi parmi les acides inorganiques tels que l'acide chlorhydrique, l'acide sulfurique, les acides perhalohydriques, tel que l'acide perchlorique, et leurs mélanges. En variante, il peut être choisi parmi les acides organiques, tels que les acides alkyl sulfuriques, par exemple l'acide décyl ou lauryl sulfurique ; les acides arylsulfoniques, tels que l'acide benzène sulfonique, l'acide paratoluène sulfonique; les acides alkylsulfoniques, tels que l'acide triflique, l'acide méthanesulfonique, l'acide éthanesulfonique, l'acide décylsulfonique, l'acide laurylsulfonique, l'acide camphosulfonique ; l'acide sulfosuccinique ; et leurs mélanges. On peut également utiliser des acides de Lewis. De préférence, il s'agit d'un acide alkylsulfonique et en particulier de l'acide méthanesulfonique, dans la mesure où il est facilement biodégradable.

Au cours de l'estérification, la fonction acide de la bétaïne salifiée est mise à réagir avec un alcool gras, pour conduire à un ester de glycine bétaïne sous forme de sel. Par "alcool gras", on entend un alcool linéaire ou ramifié (de préférence linéaire), saturé ou insaturé, comprenant de 8 à 24 atomes de carbone. Des exemples de tels alcools gras peuvent être choisis dans le groupe constitué par : l'alcool caprylique (C8:0), l'alcool décylique (C10:0), l'alcool undécylique (C11:0), l'alcool laurique (C12:0), l'alcool myristique (C14:0), l'alcool cétylique (C16:0), l'alcool palmitoléique (C16:1), l'alcool stéarique (C18:0), l'alcool oléique (C18:1), l'alcool linoléique (C18:2), l'alcool linolénique (C18:3), l'alcool arachidique (C20:0), l'alcool arachidonique (C20:4), l'alcool béhénique (C22:0), le 2-butyloctanol, le 2-hexyldécanol, le 2-octyldodécanol, le 2-décyltétradécanol et leurs mélanges. Des mélanges d'alcools gras utilisables peuvent être produits à partir d'une ou plusieurs huiles végétales et notamment d'huile de soja, d'olive, de tournesol, de maïs, de palme, de coprah, de coton, de lin, de germe de blé, de carthame ou de colza, par exemple.

La réaction d'estérification s'effectue généralement en l'absence de solvant. L'eau produite lors de la réaction contribue par ailleurs à la solubilisation de la glycine bétaïne dans le mélange réactionnel.

Pour la mise en oeuvre de cette réaction, on peut utiliser de 0,8 à 2 équivalents, par exemple de 0,9 à 1,0 équivalent, ou en variante de 1,1 à 1,8 équivalent, préférentiellement dans ce cas de 1,2 à 1,6 équivalent et, mieux, de 1,3 à 1,5 équivalent d'alcool gras. On utilise en tout état de cause de 1,5 à 1,9 équivalents, préférentiellement de 1,5 à 1,7 équivalent molaire d'acide organique ou inorganique, pour 1 équivalent de glycine bétaïne. L'estérification est réalisée à une température de 150 à 180°C. Dans une forme d'exécution préférée de l'invention, la glycine bétaïne, l'alcool gras et l'acide organique ou inorganique sont mélangés à une température de 160 à 180°C, par exemple de 170°C, puis la température du mélange est abaissée de 10 à 20°C, jusqu'à 140-160°C, par exemple à 150°C, avant le début de la réaction. La réaction peut être réalisée sous pression atmosphérique ou de préférence sous pression réduite, par exemple à une pression de 10 à 600 mbar. La pression sera généralement d'autant plus faible que la longueur de chaîne de l'alcool gras mis en jeu est grande. L'homme de l'art saura en tout état de cause ajuster la pression choisie de manière à éliminer l'eau formée lors de la réaction et à déplacer l'équilibre vers la formation de l'ester. Le milieu réactionnel est ensuite refroidi à une température de 20 à 90°C.

On récupère alors la composition tensioactive ainsi obtenue, qui renferme au moins 65% en poids, généralement de 65 à 85% en poids, par exemple de 70 à 80% en poids, d'au moins un sel d'ester de glycine bétaïne de formule Xⁿ⁻[(CH₃)₃N⁺-CH₂-COOR]ₙ où : X est un anion organique ou inorganique, R est un radical alkyle correspondant à l'alcool gras R-OH mis en oeuvre dans la réaction d'estérification, et n vaut 1 ou 2.

L'anion X est issu de l'acide XH décrit précédemment et peut donc en particulier être un chlorure, un sulfate, un perchlorate, un ion alkylsulfate, notamment décylsulfate ou laurylsulfate, un ion arylsulfonate, notamment benzène sulfonate, paratoluène sulfonate, un ion alkylsulfonate, notamment triflate, méthanesulfonate, éthanesulfonate, décylsulfonate, laurylsulfonate, camphosulfonate, ou un ion sulfosuccinate. On préfère selon l'invention que X soit choisi parmi les alkylsulfonates et les arylsulfonates, en particulier parmi les ions méthanesulfonate, triflate, paratoluènesulfonate et camphosulfonate. Il s'agit avantageusement de l'ion méthanesulfonate.

Le radical R peut de son côté être choisi parmi les groupements octyle (C8:0), décyle (C10:0), undécyle (C11:0), lauryle (C12:0), myristyle (C14:0), cétyle (C16:0), palmitoléyle (C16:1), stéaryle (C18:0), oléyle (C18:1), linoléyle (C18:2), linolényle (C18:3), arachidyle (C20:0), arachidonyle (C20:4), béhényle (C22:0), 2-butyloctyle, 2-hexyldécyle, 2-octyldodécyle et 2-décyltétradécyle.

Il est bien entendu que, dans le cas où plusieurs alcools gras sont mis en oeuvre dans la réaction d'estérification, la composition tensioactive obtenue selon l'invention comprendra plusieurs sels d'esters de glycine bétaïne. L'expression "un sel d'ester de glycine bétaïne" doit donc être comprise, dans le contexte de cette description et sauf indication contraire, comme se référant à un ou plusieurs de ces sels.

Plus précisément, la composition tensioactive obtenue selon l'invention renferme :
(a) de 65 à 85% en poids, de préférence de 70 à 80% en poids, de sel d'ester de glycine bétaïne,
(b) de 1 à 20% en poids, par exemple de 1 à 9% en poids ou de 10 à 20% en poids, d'alcool gras,
(c) de 1 à 20% en poids, par exemple de 5 à 15% en poids d'acide organique ou inorganique, et
(d) de 1 à 20% en poids, par exemple de 2 à 15% en poids, de sel de glycine bétaïne, par rapport au poids total de ces quatre constituants.

Avantageusement, ces constituants représentent au total de 90 à 100%, et de préférence de 95 à 100% du poids de la composition. Le reste des constituants de la composition peut en particulier être constitué d'un éther d'alcool gras, de formule R-O-R où R est tel que défini précédemment.

La composition tensioactive obtenue selon l'invention peut en particulier comprendre les constituants ci-dessus dans les proportions suivantes :
- dans le cas où l'alcool gras est l'alcool laurique : de 70 à 80% en poids de sel d'ester laurique de glycine bétaïne, de 0,1 à 15% en poids d'alcool laurique, de 10 à 15% en poids d'acide organique ou inorganique et de 1 à 10% en poids de sel de glycine bétaïne ;
- dans le cas où l'alcool gras est un mélange d'alcools laurique et myristique : de 65 à 75%, de préférence de 70 à 75% en poids de sels d'esters laurique et myristique de glycine bétaïne, de 1 à 20%, par exemple de 1 à 9% en poids ou de 10 à 20% en poids d'alcools laurique et myristique, de 10 à 20%, de préférence de 10 à 15% en poids d'acide organique ou inorganique et de 1 à 15% en poids de sel de glycine bétaïne ;
- dans le cas où l'alcool gras est l'alcool oléique : de 70 à 80%, de préférence de 70 à 75% en poids de sel d'ester oléique de glycine bétaïne, de 1 à 20%, par exemple de 5 à 10% en poids ou de 11 à 20% en poids d'alcool oléique, de 5 à 15% en poids d'acide organique ou inorganique et de 1 à 15% en poids de sel de glycine bétaïne.

Il a été observé que l'utilisation d'alcool en défaut, par rapport à la glycine bétaïne, dans le procédé selon l'invention permettait d'obtenir des compositions tensioactives qui, outre leur plus faible tension de surface, par comparaison avec les compositions de l'art antérieur, présentaient une meilleure solubilité dans l'eau. Les solutions aqueuses tensioactives obtenues à partir de ces compositions tensioactives sont ainsi plus fluides et plus transparentes, ce qui est avantageux dans le cas où la solution est destinée à être pulvérisée ou imprégnée sur un support fibreux tel que des lingettes.

### Utilisations

La composition tensioactive obtenue selon l'invention présente une valeur de tension de surface inférieure à 30 mN/m, voire inférieure à 25 mN/m et généralement supérieure à 20 mN/m, mesurée selon la norme NF EN 14370.

Il est ainsi possible d'envisager son utilisation dans une diversité d'applications comme agent mouillant, dispersant de particules, inhibiteur de corrosion, solubilisant, antistatique, démêlant et/ou pour améliorer l'effet et/ou la rémanence de substances insecticides et/ou pour améliorer le pouvoir désinfectant de substances antimicrobiennes. Elle peut en particulier être utilisée pour la fabrication de plastiques ou de différents produits destinés notamment :
- au traitement et/ou au nettoyage du corps, de plantes, de textiles ou de surfaces dures, en particulier de produits cosmétiques, tels que des shampooings, après-shampooings, savons liquides, bains moussants et gels-douches ; de produits de lavage de véhicules tels que des automobiles, des camions, des trains, des bus ou des avions ; de produits ménagers tels que des détergents pour les vitres, les surfaces murales, les sols ou la vaisselle ; de produits d'entretien pour les textiles ; de produits de nettoyage industriel ; de produits d'ensimage de fibres ; de produits phytosanitaires ; de produits pigmentés tels que des peintures ou vernis ;
- au traitement de l'eau ;
- au revêtement de routes, par exemple d'émulsions bitumineuses ;
- à l'extraction du pétrole.

Dans le cas du traitement de l'eau, la composition selon l'invention permet de décoller le biofilm sans détruire l'efficacité des résines échangeuses d'ions, contrairement aux tensioactifs cationiques classiques qui présentent par ailleurs un impact environnemental non négligeable compte tenu de leur absence de biodégradabilité, ou de leur biodégradabilité plus lente. Cette capacité à décoller les biofilms peut également être mise à profit dans les procédés d'extraction de pétrole.

Dans des applications cosmétiques, la composition selon l'invention est compatible avec les tensioactifs anioniques classiques et permet d'améliorer le caractère crémeux de la mousse qu'ils génèrent. Elle protège également les dispositifs aérosols en fer contre la corrosion. Il a en outre été démontré que la composition selon l'invention permet de réduire l'électricité statique des cheveux et facilite leur démêlage. Le pouvoir solubilisant de la composition selon l'invention permet également d'envisager son utilisation pour la solubilisation de parfums ou d'huiles essentielles et sa capacité dispersante peut permettre de disperser les pigments présents dans les compositions cosmétiques.

Dans la fabrication de plastiques, la composition selon l'invention permet de conférer des propriétés électrostatiques à la surface du plastique, sans affecter ses capacités de recyclage compte tenu de son caractère biosourcé.

Lorsqu'elle est utilisée dans la fabrication de produits phytosanitaires, la composition selon l'invention permet d'améliorer la rémanence des matières actives et la résistance à l'eau des produits tels que des herbicides, des pesticides ou des agents modifiant la croissance des plantes, qui peuvent ainsi être utilisés en plus faible quantité. Cette composition peut ainsi être ajoutée, sous forme diluée à 25% dans l'eau, à raison de 0,4% en poids, dans un produit renfermant un milieu acide, par exemple.

La composition selon l'invention peut par ailleurs être utilisée dans un procédé d'extraction, de stockage, d'entreposage ou de raffinage de pétrole pour limiter la corrosion des équipements. Dans cette application, elle peut être ajoutée au pétrole à hauteur de 500 à 1000 ppm, par exemple.

L'invention a également pour objet un produit, par exemple choisi parmi ceux décrits ci-dessus, comprenant une composition selon l'invention et au moins un composé choisi parmi : les tensioactifs anioniques, les tensioactifs non ioniques, les agents anti-microbiens et leurs mélanges. Des exemples de tensioactifs anioniques sont : les sels de sulfate d'alcools gras éthoxylés, les sulfosuccinates, les sarcosinates, les alkyl- et dialkylphosphates, les savons d'acides gras et leurs mélanges. Les tensioactifs non ioniques peuvent par exemple être choisis parmi : les esters d'acides gras et de polyols tel que les esters d'acides gras et de glycérol éventuellement polyéthoxylés, les esters d'acides gras et de sorbitane éventuellement polyéthoxylés, les esters d'acides gras et de polyoxyéthylène, les esters d'acides gras et de sucrose comme le stéarate de sucrose, les éthers d'alcool gras et de polyoxyéthylène, les éthers d'alcools gras et de sucre, notamment les alkylpolyglucosides (APG), les polysiloxanes modifiés polyéthers, et leurs mélanges. Les agents anti-microbiens peuvent être choisis parmi les ammoniums quaternaires, les aldéhydes (tels que le glutaraldéhyde et le formaldéhyde), l'éthanol, les dérivés halogénés, les oxydants, les composés phénoliques, les parabens, les isothiazolones (ou isothiazolinones), les benzoates, l'imidazoline, l'hydantoïne, la guanidine, les acides organiques tels que l'acide lactique, et leurs mélanges. Les substances insecticides peuvent être choisies parmi les agents organosphosphorés (tels que l'acéphate, le chlorpyrifos ou le bromophos), les nicotinoïdes, les pyréthrinoïdes (tels que la perméthrine, la bifenthrine ou la fenvalérate), les monoterpènes (tels que le p-menthane-3,8-diol), les composés organohalogénés (tels que le lindane, le dicofol ou le toxaphène), le N,N-diéthyl-3-méthylbenzamide, les dérivés du pyrèthre (tels que la Pyréthrine I, la Pyréthrine II ou la Jasmoline I), les sulfones, les sulfonates, les formamidines, les benzoylurées, les roténones, les alcaloïdes, la quassine, la ryanidone, l'aconitine, le géraniol et leurs mélanges.

Ce produit se présente avantageusement sous forme de solution aqueuse ou de gel aqueux. En variante, il peut se présenter sous forme d'émulsion huile-dans-eau ou eau-dans-huile voire de pâte. En tout état de cause, la phase aqueuse contenue dans ce produit présente avantageusement un pH allant de 1 à 8, notamment de 1 à 5. Ce produit peut être conditionné dans tout dispositif adapté à l'utilisation envisagée et notamment dans un flacon-pompe, un tube, un pot, un dispositif aérosol ou une lingette.

Il renferme avantageusement de 0,1 à 25% en poids, par exemple de 1 à 10% en poids, de composition tensioactive selon l'invention.

Le produit selon l'invention peut également comprendre, en plus des agents anti-microbiens, insecticides et tensioactifs précités, et suivant l'application envisagée, au moins un ingrédient choisi parmi : les actifs phytosanitaires ou cosmétiques, les enzymes, les agents chélatants, les épaississants, les corps gras (huiles, cires et/ou pâteux), les charges, les conservateurs, les pigments et colorants, les anti-oxydants, les azurants optiques, et leurs mélanges.

### FIGURES

La Figure 1 illustre la peignabilité d'une mèche de cheveux traitée respectivement avec de l'eau, une composition renfermant un tensioactif de référence et une même composition renfermant un mélange de sels d'esters de glycine bétaïne selon l'invention.
La Figure 2 est un diagramme illustrant la douceur de mèches traitées à l'aide d'une composition démêlante renfermant un tensioactif de référence ou un mélange de sels d'esters de glycine bétaïne selon l'invention, par comparaison avec un démêlant du commerce.

### EXEMPLES

L'invention sera mieux comprise à la lumière des exemples suivants, qui sont donnés à titre purement illustratif et n'ont pas pour but de limiter la portée de l'invention, définie par les revendications annexées.

### Exemple 1 : Synthèse d'une composition tensioactive selon l'invention

De la glycine bétaïne (1,0 éq) et un alcool gras sont introduits dans un réacteur. La température de consigne au niveau du mélange est fixée à 170°C et la pression est réduite jusqu'à une valeur de 10 à 600 mbar, suivant l'alcool utilisé. Une fois les consignes de pression et de température atteintes, une solution d'acide méthanesulfonique 70 % (1,6 éq) est ajoutée au mélange réactionnel. Dès que l'addition est terminée, la température de consigne est ramenée à 150°C et la pression est maintenue à une valeur de 10 à 600 mbar, suivant l'alcool utilisé. Quatre heures après le début de l'introduction de l'acide, on laisse refroidir le mélange réactionnel à 80°C, puis le produit est récupéré, refroidi jusqu'à température ambiante, et le rendement de la réaction est déterminé par RMN ¹H.

Ce procédé a été mis en oeuvre en utilisant différents alcools gras. Ceux-ci ont été utilisés soit en excès (1,4 équivalent), soit en défaut (0,9 équivalent) par rapport à la glycine bétaïne.

La mesure de tension superficielle a été réalisée selon la norme NF EN 14370, à l'aide d'un tensiomètre Krüss avec un anneau en platine suspendu horizontalement. Avant chaque mesure, l'anneau est minutieusement nettoyé et séché à la flamme. Le godet pour échantillon est un récipient conique en PTFE placé dans une enceinte thermorégulée à 25°C. L'échantillon est préparé avec de l'eau Milli-Q et continuellement agité à l'aide d'un barreau magnétique avant chaque mesure.

Les produits obtenus présentaient la composition massique suivante :

| | Alcool en excès | | | Alcool en défaut | | |
|---|---|---|---|---|---|---|
| Constituant | R=C12:0 | R=C12:0/ C14:0 | R=C18:1 | R=C12:0 | R=C12:0 / C14:0 | R=C18:1 |
| Mésylate de bétaïnate d'alkyle | 74% | 72% | 72% | 77% | 70% | 72% |
| Alcool gras résiduel | 11% | 13% | 16% | 1% | 4% | 6% |
| Acide méthanesulfonique résiduel | 12% | 11% | 9% | 14% | 14% | 12% |
| Mésylate de glycine bétaïne résiduel | 3% | 4% | 3% | 8% | 12% | 10% |
| Rendement⁽¹⁾ | 93 % | 90% | 92% | 93% | 86% | 83% |
| Tension superficielle à la CMC (mN/m) | 21 à pH 3 | 21,8 à pH 3 | 29 à pH 3 | 21 à pH 3 | 21,8 à pH 3 | 29 à pH 3 |

| | Alcool en excès | |
|---|---|---|
| Constituant | R = 2-hexyldécyle | R = 2-octyldodécyle |
| Mésylate de bétaïnate d'alkyle | 69% | 73 % |
| Alcool gras résiduel | 17 % | 16 % |
| Acide méthanesulfonique résiduel | 11 % | 10 % |
| Mésylate de glycine bétaïne résiduel | 3 % | 1 % |
| Rendement | 93 % | 96% |
| Tension superficielle à la CMC (mN/m) | 26,0 mN/m à pH = 3 | 25,9 mN/m à pH = 3 |

| | Alcool en excès | Alcool en défaut |
|---|---|---|
| Constituant | R = C10:0 | R = C10:0 |
| Mésylate de bétaïnate d'alkyle | 71 % | 75 % |
| Alcool gras résiduel | 14 % | 2% |
| Acide méthanesulfonique résiduel | 12 % | 14 % |
| Mésylate de glycine bétaïne résiduel | 4% | 9% |
| Rendement | 92% | 91 % |
| Tension superficielle à la CMC (mN/m) | 23,0 mN/m à pH = 3 | 26,3 mN/m à pH = 3 |

| | | |
|---|---|---|
| ⁽¹⁾ Calculé par rapport à la glycine bétaïne (alcool en excès) ou à l'alcool gras (alcool en défaut) | | |

On observe que le rendement de la réaction est toujours supérieur ou égal à 75%. Par ailleurs, pour les esters renfermant au moins 12 atomes de carbone, la tension de surface à la CMC des compositions obtenues selon l'invention ne dépend pas de la quantité d'alcool gras mise en oeuvre, ce qui permet d'utiliser une plus faible quantité d'alcool. Dans la mesure où ce dernier impacte significativement le coût du procédé, il s'agit d'un avantage économique indéniable du procédé selon l'invention. En outre, l'alcool gras résiduel réduisant la solubilité dans l'eau des compositions tensioactives, l'utilisation d'alcool en défaut dans le procédé selon l'invention permet d'obtenir des solutions aqueuses tensioactives plus fluides et transparentes à partir de ces compositions tensioactives.

Par comparaison, les produits décrits dans les documents WO 2013/188508 et US-7,829,521 et dans la publication de Goursaud et al (Green Chem., 2008, 10, 310-320) présentaient les caractéristiques suivantes :

| Constituant | R=C12:0 | | R=C 12: 0/ C14:0 | R=C18:1 | | R=C18:0 | |
|---|---|---|---|---|---|---|---|
| | Goursaud | WO'508 | WO'508 | US'521 | Goursaud | US'521 | Goursaud |
| Rendement | 95% | -- | -- | 85% | 85% | 80% | 70% |
| Tension superficielle à la CMC (mN/m) | 32 | 27 | 24 | 36,6 | 37 | 37 | 37 |

Ces produits ont été obtenus, d'après ces documents, en faisant réagir la glycine bétaïne avec de 1,0 à 1,5 équivalent d'alcool en présence de 2 à 3 équivalents d'acide méthanesulfonique, à une température de 120 à 140°C et sous une pression de 30 à 100 mbar, pendant une durée de réaction de 6 à 8h. Les produits obtenus par Goursaud et dans US'521 ont par ailleurs été purifiés.

Il apparaît ainsi que les produits obtenus selon l'invention, en utilisant une plus faible quantité d'acide méthanesulfonique et une température plus élevée, présentent, de manière totalement inattendue, des propriétés améliorées, permettant d'envisager leur utilisation dans une diversité d'applications. Ces produits sont par ailleurs obtenus avec un rendement du même ordre de grandeur (supérieur à 80%) que ceux de l'art antérieur mais en un temps plus court, ce qui représente un avantage indéniable du point de vue industriel.

### Exemple 2 : Test de démêlage

On a réalisé un essai comparatif de démêlage d'une mèche de cheveux à l'aide d'une émulsion d'alcool gras dans l'eau renfermant soit un mélange de sels d'esters de glycine bétaïne en C18 à C22 selon l'invention, soit du chlorure de behentrimonium (Varisoft^{®} BT 85 d'EVONIK). De l'eau a par ailleurs été utilisée comme témoin. Pour ce faire, 2 mèches de cheveux ont été préalablement lavées à l'aide d'une solution à base de lauryl éther sulfate, rincées à l'eau puis essorées et enfin frottées 15 fois dans la paume de la main pour emmêler les cheveux. 1g de chaque produit a ensuite été appliqué sur l'une des deux mèches humides qui a alors été massée 8 fois sur toute sa longueur pour bien répartir le produit. Après 3 minutes de temps de pose, les mèches ont été rincées à l'eau de ville puis essorées à la main. Après les avoir déposées sur une surface plane, on a mesuré le nombre de coups de peigne nécessaires pour obtenir une mèche peignable sans contrainte. Cette procédure a été répétée trois fois par produit, sur 3 mèches différentes. Un seul essai a été réalisé avec l'eau de ville.

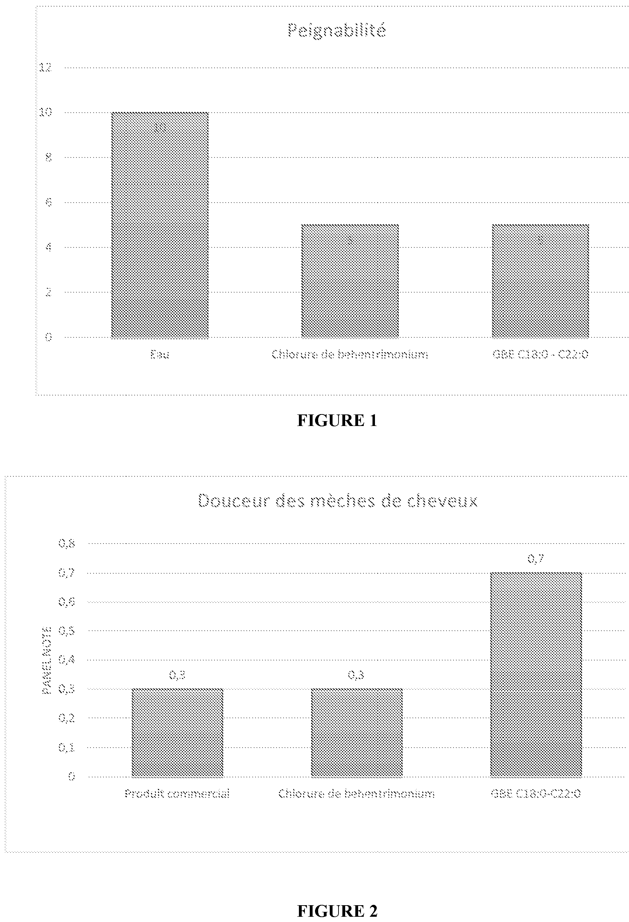

Les résultats de ces essais sont illustrés à la Figure 1. Comme il ressort de cette Figure, les sels d'esters de glycine bétaïne selon l'invention offrent des performances équivalentes au tensioactif de référence. Les dérivés de glycine bétaïne présentent toutefois une biodégradabilité supérieure au tensioactif de référence, sont biosourcés à 100% et leur procédé de synthèse est plus respectueux de l'environnement.

Une analyse sensorielle de la douceur des mèches ainsi obtenues a ensuite été réalisée par trois panélistes, par comparaison avec une mèche traitée de la même manière que ci-dessus, mais avec un produit démêlant du commerce (Elsève^{®} Total Repair Rapid Restore). Les résultats de cette évaluation sont présentés sur la Figure 2. Comme le montre cette Figure, les mèches obtenues sont perçues comme beaucoup plus douces que celles traitées avec le tensioactif de référence et même avec le démêlant du commerce.

### Exemple 3 : Formulations

Plusieurs types de produits peuvent être préparés à l'aide de compositions tensioactives selon l'invention, à base respectivement d'ester laurique (GBE C12:0), d'estermyristique (GBE C14:0), d'ester palmitique (GBE C16:0), d'ester stéarique (GBE C18:0), d'ester oléique (GBE C18:1), d'ester béhénylique (GBE C22:0), d'ester 2-octyldodécylique (GBE C20R:0) ou de leurs mélanges.

### Détergent ménager

| | | |
|---|---|---|
| Acide lactique 80% | | 2,00 % |
| GBE C12:0/C14:0 | | 0,40 % |
| Hydroxyéthyl cellulose | | 0,30 % |
| Chélatant | | 0,20 % |
| Parfum | | 0,20 % |
| Colorant | | 0,01 % |
| Eau désionisée | qsp | 100,00 % |

### Adoucissant concentré

| | | |
|---|---|---|
| GBE C20R:0 | | 15,00 % |
| Chlorure de calcium à 25% | | 1,50 % |
| Parfum | | 0,70 % |
| Colorant | | 0,02 % |
| Conservateur | | 0,10 % |
| Eau | sp | 100,00 % |

### Produit WC

| | | |
|---|---|---|
| Acide phosphorique 85% | | 5,90 % |
| Acide chlorhydrique 37% | | 13,50 % |
| GBE C12:0 | | 3,00 % |
| Parfum | | 0,20 % |
| Colorant | | 0,01 % |
| Eau | qsp | 100,00 % |

### Shampooing

| | | |
|---|---|---|
| GBE C18:1 | | 30,00 % |
| Diéthanolamide de coprah | | 5,00 % |
| Acide citrique | | qsp pH 5 |
| Conservateur | | qs |
| Eau | qsp | 100,00 % |

### Après-shampooing

| | | |
|---|---|---|
| GBE C16:0/C18:0 | | 3,00 % |
| Alcools cétylique / stéarylique | | 5,00 % |
| Citrate de sodium | qs | pH 2,5-3,0 |
| Parfum | | 0,25 % |
| Colorant | | 0,02 % |
| Conservateur | | 0,01 % |
| Eau | qsp | 100,00 % |

### Masque capillaire

| | | |
|---|---|---|
| GBE C18:0/C22:0 | | 3,00 % |
| Alcools cétylique / stéarylique | | 5,00 % |
| Huile d'olive | | 5,00 % |
| Citrate de sodium | qs | pH 2,5-3,0 |
| Panthénol | | 0,15 % |
| Parfum | | 0,05 % |
| Colorant | | 0,02 % |
| Conservateur | | 0,01 % |
| Eau | qsp | 100,00 % |

### Adjuvant phytosanitaire (concentré émulsionnable)

| | |
|---|---|
| GBE C12:0 | 6,00 % |
| GBE C18:1 | 8,00 % |
| Agent anti-mousse | 0,10 % |
| Ester méthylique de colza qsp | 100,00 % |

## Revendications

1. Procédé de préparation d'une composition tensioactive, comprenant les étapes successives consistant à :
1) faire réagir de la glycine bétaïne ou l'un de ses sels avec un alcool gras linéaire ou ramifié, saturé ou insaturé, renfermant de 8 à 24 atomes de carbone, en présence d'un acide organique ou inorganique ;
2) refroidir le milieu réactionnel à une température de 20 à 90°C ; et
3) récupérer la composition tensioactive ainsi obtenue,
**caractérisé en ce que** l'acide organique ou inorganique représente de 1,5 à 1,9 équivalent molaire par rapport au nombre de moles de glycine bétaïne et **en ce que** la réaction est effectuée à une température de 150 à 180°C.

2. Procédé selon la revendication 1, **caractérisé en ce que** l'acide est choisi parmi : les acides inorganiques tels que l'acide chlorhydrique, l'acide sulfurique, les acides perhalohydriques, tel que l'acide perchlorique, et leurs mélanges ; les acides organiques, tels que les acides alkyl sulfuriques, par exemple l'acide décyl ou lauryl sulfurique, les acides arylsulfoniques, tels que l'acide benzène sulfonique, l'acide paratoluène sulfonique, les acides alkylsulfoniques, tels que l'acide triflique, l'acide méthanesulfonique, l'acide éthanesulfonique, l'acide décylsulfonique, l'acide laurylsulfonique, l'acide camphosulfonique, l'acide sulfosuccinique, et leurs mélanges, de préférence l'acide est l'acide méthanesulfonique.

3. Procédé selon la revendication 1 ou 2, **caractérisé en ce que** l'alcool gras est choisi dans le groupe constitué par : l'alcool caprylique (C8:0), l'alcool décylique (C10:0), l'alcool undécylique (C11:0), l'alcool laurique (C12:0), l'alcool myristique (C14:0), l'alcool cétylique (C16:0), l'alcool palmitoléique (C16:1), l'alcool stéarique (C18:0), l'alcool oléique (C18:1), l'alcool linoléique (C18:2), l'alcool linolénique (C18:3), l'alcool arachidique (C20:0), l'alcool arachidonique (C20:4), l'alcool béhénylique (C22:0), le 2-butyloctanol, le 2-hexyldécanol, le 2-octyldodécanol, le 2-décyltétradécanol et leurs mélanges.

4. Procédé selon l'une quelconque des revendications 1 à 3, **caractérisé en ce qu'**on utilise de 0,8 à 2 équivalents, par exemple de 0,9 à 1,0 équivalent, ou en variante de 1,1 à 1,8 équivalent, préférentiellement dans ce cas de 1,2 à 1,6 équivalent et, mieux, de 1,3 à 1,5 équivalent d'alcool gras, pour 1 équivalent de glycine bétaïne.

5. Procédé selon l'une quelconque des revendications 1 à 4, **caractérisé en ce qu'**on utilise de 1,5 à 1,7 équivalent molaire d'acide organique ou inorganique, pour 1 équivalent de glycine bétaïne.

6. Composition tensioactive obtenue suivant le procédé selon l'une quelconque des revendications 1 à 5, **caractérisée en ce qu'**elle renferme les constituants suivants :
a) de 65 à 85% en poids, de préférence de 70 à 80% en poids, de sel d'ester de glycine bétaïne,
b) de 1 à 20% en poids, par exemple de 1 à 9% en poids ou de 10 à 20% en poids, d'alcool gras,
c) de 1 à 20% en poids, par exemple de 5 à 15% en poids d'acide organique ou inorganique, et
d) de 1 à 20% en poids, par exemple de 2 à 15% en poids, de sel de glycine bétaïne,
par rapport au poids total de ces quatre constituants.

7. Composition selon la revendication 6, caractérisée en ces constituants représentent au total de 90 à 100%, et de préférence de 95 à 100% du poids de la composition.

8. Utilisation de la composition tensioactive selon l'une quelconque des revendications 6 à 7 comme agent mouillant, dispersant de particules, inhibiteur de corrosion, solubilisant, antistatique et/ou démêlant et/ou pour améliorer l'effet et/ou la rémanence de substances insecticides et/ou pour améliorer le pouvoir désinfectant et/ou la rémanence de l'effet désinfectant de substances antimicrobiennes.

9. Utilisation de la composition selon l'une quelconque des revendications 6 à 7 pour la fabrication de plastiques ou de produits destinés :
• au traitement et/ou au nettoyage du corps, de plantes ou de surfaces dures, en particulier de produits cosmétiques, de produits de lavage de véhicules, de produits ménagers, de produits de nettoyage industriel, de produits d'ensimage de fibres et de produits phytosanitaires ;
• au traitement de l'eau ;
• au revêtement de routes ; ou
• à l'extraction du pétrole.

10. Produit comprenant la composition selon l'une quelconque des revendications 6 à 7 et au moins un constituant choisi parmi : (a) les tensioactifs anioniques, les tensioactifs non ioniques et leurs mélanges, (b) les agents antimicrobiens, (c) les insecticides, et leurs mélanges.

## Patentansprüche

1. Verfahren zur Herstellung einer Tensidzusammensetzung, das die folgenden aufeinanderfolgenden Schritte umfasst, die aus Folgendem bestehen:
1) dem Umsetzen von Glycinbetain oder einem Salz davon mit einem gesättigten oder ungesättigten, linearen oder verzweigten Fettalkohol, der 8 bis 24 Kohlenstoffatome umfasst, in Gegenwart einer organischen oder anorganischen Säure;
2) dem Abkühlen des Reaktionsmediums auf eine Temperatur von 20 bis 90 °C und
3) dem Isolieren der so erhaltenen Tensidzusammensetzung,
**dadurch gekennzeichnet, dass** die organische oder anorganische Säure 1,5 bis 1,9 Moläquivalente, bezogen auf die Stoffmenge von Glycinbetain, darstellt und dass die Reaktion bei einer Temperatur von 150 bis 180 °C durchgeführt wird.

2. Verfahren nach Anspruch 1, **dadurch gekennzeichnet, dass** die Säure ausgewählt ist aus: anorganischen Säuren, wie Salzsäure, Schwefelsäure, Perhalogensäuren, wie Perchlorsäure, und Mischungen davon; organische Säuren, wie Alkylschwefelsäuren, beispielsweise Decyl- oder Laurylschwefelsäure, Arylsulfonsäuren, wie Benzolsulfonsäure, Paratoluolsulfonsäure, Alkylsulfonsäuren, wie Trifluormethansulfonsäure, Methansulfonsäure, Ethansulfonsäure, Decylsulfonsäure, Laurylsulfonsäure, Camphersulfonsäure, Sulfobernsteinsäure, und Mischungen davon, wobei die Säure vorzugsweise Methansulfonsäure ist.

3. Verfahren nach Anspruch 1 oder 2, **dadurch gekennzeichnet, dass** der Fettalkohol ausgewählt ist aus der Gruppe bestehend aus: Caprylalkohol (C8:0), Decylalkohol (C10:0), Undecylalkohol (C11:0), Laurylalkohol (C12:0), Myristinalkohol (C14:0), Cetylalkohol (C16:0), Palmitoleinalkohol (C16:1), Stearylalkohol (C18:0), Oleinalkohol (C18:1), Linoleylalkohol (C18:2), Linolenalkohol (C18:3), Arachidylalkohol (C20:0), Arachidonalkohol (C20:4), Behenylalkohol (C22:0), 2-Butyloctanol, 2-Hexyldecanol, 2-Octyldodecanol, 2-Decyltetradecanol und Mischungen davon.

4. Verfahren nach einem der Ansprüche 1 bis 3, **dadurch gekennzeichnet, dass** 0,8 bis 2 Äquivalente, beispielsweise 0,9 bis 1,0 Äquivalente, oder alternativ 1,1 bis 1,8 Äquivalente, vorzugsweise in diesem Fall 1,2 bis 1,6 Äquivalente und noch mehr bevorzugt 1,3 bis 1,5 Äquivalente Fettalkohol auf 1 Äquivalent Glycinbetain verwendet werden.

5. Verfahren nach einem der Ansprüche 1 bis 4, **dadurch gekennzeichnet, dass** 1,5 bis 1,7 Moläquivalente einer organischen oder anorganischen Säure auf 1 Äquivalent Glycinbetain verwendet werden.

6. Tensidzusammensetzung, die gemäß dem Verfahren nach einem der Ansprüche 1 bis 5 erhalten wird, **dadurch gekennzeichnet, dass** sie die folgenden Bestandteile umfasst:
a) 65 bis 80 Gew.-%, vorzugsweise 70 bis 80 Gew.-%, eines Glycinbetainestersalzes,
b) 1 bis 20 Gew.-%, beispielsweise 1 bis 9 Gew.-% oder 10 bis 20 Gew.-%, eines Fettalkohols,
c) 1 bis 20 Gew.-%, beispielsweise 5 bis 15 Gew.-%, einer organischen oder anorganischen Säure und
d) 1 bis 20 Gew.-%, beispielsweise 2 bis 15 Gew.-%, eines Glycinbetainsalzes,
bezogen auf das Gesamtgewicht dieser vier Bestandteile.

7. Zusammensetzung nach Anspruch 6, **dadurch gekennzeichnet, dass** diese Bestandteile insgesamt 90 bis 100 % und vorzugsweise 95 bis 100 % des Gewichts der Zusammensetzung darstellen.

8. Verwendung der Tensidzusammensetzung nach einem der Ansprüche 6 bis 7 als Benetzungsmittel, Dispergiermittel für Partikel, Korrosionsinhibitor, Lösungsvermittler, Antistatikum und/oder Entwirrungsmittel und/oder zur Verbesserung der Wirkung und/oder der Persistenz von insektiziden Substanzen und/oder zur Verbesserung des Desinfektionsvermögens und/oder der Persistenz der desinfizierenden Wirkung von antimikrobiellen Substanzen.

9. Verwendung der Zusammensetzung nach einem der Ansprüche 6 bis 7 zur Herstellung von Kunststoffen oder von Produkten, die vorgesehen sind:
- zur Behandlung und/oder Reinigung des Körpers, von Pflanzen oder harten Oberflächen, insbesondere für Kosmetika, Produkte zur Fahrzeugreinigung, Haushaltsprodukte, Industriereiniger, Produkte zum Schlichten von Fasern und Pflanzenschutzmittel;
• zur Wasseraufbereitung;
• für Straßenbeläge oder
• zur Erdölgewinnung.

10. Produkt, das die Zusammensetzung nach einem der Ansprüche 6 bis 7 und mindestens einen Bestandteil umfasst, der ausgewählt ist aus: (a) anionischen Tensiden, nichtionischen Tensiden und Mischungen davon, (b) antimikrobiellen Substanzen, (c) Insektiziden und Mischungen davon.

## Claims

1. A process for preparing a surfactant composition, comprising the successive steps consisting in:
1) reacting glycine betaine or a salt thereof with a saturated or unsaturated, linear or branched fatty alcohol containing from 8 to 24 carbon atoms, in the presence of an organic or inorganic acid;
2) cooling the reaction medium to a temperature of from 20 to 90°C; and
3) collecting the surfactant composition thus obtained,
**characterized in that** the organic or inorganic acid represents from 1.5 to 1.9 molar equivalents relative to the number of moles of glycine betaine and **in that** the reaction is performed at a temperature of from 150 to 180°C.

2. The process as claimed in claim 1, **characterized in that** the acid is chosen from: inorganic acids such as hydrochloric acid, sulfuric acid, perhydrohalic acids, such as perchloric acid, and mixtures thereof; organic acids, such as alkylsulfuric acids, for example decyl or laurylsulfuric acid, arylsulfonic acids, such as benzenesulfonic acid, para-toluenesulfonic acid, alkylsulfonic acids, such as triflic acid, methanesulfonic acid, ethanesulfonic acid, decylsulfonic acid, laurylsulfonic acid, camphorsulfonic acid, sulfosuccinic acid, and mixtures thereof; preferably, the acid is methanesulfonic acid.

3. The process as claimed in claim 1 or 2, **characterized in that** the fatty alcohol is chosen from the group consisting of: capryl alcohol (C8:0), decyl alcohol (C10:0), undecyl alcohol (C11:0), lauryl alcohol (C12:0), myristyl alcohol (C14:0), cetyl alcohol (C16:0), palmitoleyl alcohol (C16:1), stearyl alcohol (C18:0), oleyl alcohol (C18:1), linoleyl alcohol (C18:2), linolenyl alcohol (C18:3), arachidyl alcohol (C20:0), arachidonyl alcohol (C20:4), behenyl alcohol (C22:0), 2-butyloctanol, 2-hexyldecanol, 2-octyldodecanol, 2-decyltetradecanol and mixtures thereof.

4. The process as claimed in any one of claims 1 to 3, **characterized in that** from 0.8 to 2 equivalents, for example from 0.9 to 1.0 equivalent, or as a variant from 1.1 to 1.8 equivalents, preferentially in this case from 1.2 to 1.6 equivalents and better still from 1.3 to 1.5 equivalents of fatty alcohol, per 1 equivalent of glycine betaine, are used.

5. The process as claimed in any one of claims 1 to 4, **characterized in that** from 1.5 to 1.7 molar equivalents of organic or inorganic acid per 1 equivalent of glycine betaine are used.

6. A surfactant composition obtained according to the process as claimed in any one of claims 1 to 5, **characterized in that** it contains the following constituents:
a) from 65% to 85% by weight, preferably from 70% to 80% by weight, of a glycine betaine ester salt,
b) from 1% to 20% by weight, for example from 1% to 9% by weight or from 10% to 20% by weight, of a fatty alcohol,
c) from 1% to 20% by weight, for example from 5% to 15% by weight, of an organic or inorganic acid, and
d) from 1% to 20% by weight, for example from 2% to 15% by weight, of a glycine betaine salt,
relative to the total weight of these four constituents.

7. The composition as claimed in claim 6, **characterized in that** said constituents represent a total of from 90% to 100% and preferably from 95% to 100% by weight of the composition.

8. The use of the surfactant composition as claimed in any one of claims 6 to 7, as a wetting agent, particle dispersant, corrosion inhibitor, solubilizing agent, antistatic and/or disentangling agent and/or for improving the effect and/or the persistence of insecticidal substances and/or for improving the disinfecting power and/or the persistence of the disinfecting effect of antimicrobial substances.

9. The use of the composition as claimed in any one of claims 6 to 7, for manufacturing plastics or products intended for:
• treating and/or cleansing the body, plants or hard surfaces, in particular cosmetic products, products for washing vehicles, household products, industrial cleaning products, fiber sizing products and plant protection products;
• treating water;
• road surfacing; or
• extracting petroleum.

10. A product comprising the composition as claimed in any one of claims 6 to 7 and at least one constituent chosen from: (a) anionic surfactants, nonionic surfactants and mixtures thereof, (b) antimicrobial agents, (c) insecticides, and mixtures thereof.
